Europäisches Patentamt

European Patent Office

Office européen des brevets

(11) Publication number: **0 310 178**
**A1**

# EUROPEAN PATENT APPLICATION

(21) Application number: 88202063.9

(22) Date of filing: 22.09.88

(51) Int. Cl.4: **C12P 21/02** , **A61K 39/29** ,
**//C12N15/00**

(30) Priority: 30.09.87 US 102957
18.04.88 US 182557

(43) Date of publication of application:
05.04.89 Bulletin 89/14

(84) Designated Contracting States:
CH DE FR GB IT LI NL

(71) Applicant: MERCK & CO. INC.
126, East Lincoln Avenue P.O. Box 2000
Rahway New Jersey 07065-0900(US)

(72) Inventor: Kubek, Dennis J.
1127 Vilsmeier Road
Lansdale, PA 19446(US)

(74) Representative: Hesketh, Alan, Dr.
European Patent Department Merck & Co.,
Inc. Terlings Park Eastwick Road
Harlow Essex, CM20 2QR(GB)

(54) Recovery of pres2 + S antigen.

(57) A liquid phase containing preS2 + S antigen is separated into two phases after which the desired antigen is concentrated, washed and adsorbed and desorbed from a fumed silica to produce a product that is purified and concentrated in antigen:protein ratio and that is suitable for final purification.

EP 0 310 178 A1

## RECOVERY OF PRES2 + S ANTIGEN

## BACKGROUND OF THE INVENTION

The hepatitis B virus preS2 antigen gene linked in one contiguous reading frame to the hepatitis B virus surface antigen gene has been expressed in a transformed host. The expressed protein is useful as a vaccine for the treatment and prevention of hepatitis B virus-induced diseases and/or infections and in in vitro diagnostic systems. The purification of the expressed protein from either broken cell slurries or from cell supernatant liquids, however, has heretofore resulted in large losses in yield due to degradation of the antigen.

## OBJECTS OF THE INVENTION

It is, therefore, an object of the present invention to provide a method for the recovery of preS2 + S antigen in high yield. Another object is to provide a method that facilitates purification of the preS2 + S antigen. These and other objects of the present invention will be apparent from the following description.

## SUMMARY OF THE INVENTION

A liquid phase with or without solids containing preS2 + S antigen is separated into two aqueous phases after which the desired antigen is adsorbed and desorbed from a fumed silica to produce a product purified in 50-100 fold (antigen:protein ratio) that is suitable for final concentration and purification.

## DETAILED DESCRIPTION

The present invention relates to the .recovery and purification of preS2 + S antigen from broken cell slurries or cell supernatant liquids.

According to the present invention, the foregoing objects are achieved by a two-phase aqueous extraction of the preS2 + S antigen coupled with adsorption to and desorbtion from fumed silica.

A broken cell slurry or a cell supernatant liquid is separated into two phases by adding to the broken cell slurry or cell supernatant liquid polyethylene glycol (PEG) and dextran. The PEG and dextran form two aqueous phases and the pres2 + S antigen migrates to the upper PEG-containing layer. A surfactant preferably is present to increase the solubility of the antigen. A pH buffer and salts preferably are present to enhance the migration of the antigen into the PEG containing layer.

A suitable PEG is PEG 3350 and a suitable dextran is Dextran T500. An example of a nonionic surfactant is Triton X-100. Examples of useful salts are NaCl and $K_2HPO_4$.

The broken cell slurry or cell supernatant liquid with the foregoing added ingredients is centifuged and the top phase is drawn off, diluted and filtered.

The filtrate is concentrated and diafiltered to remove PEG. Residual surfactant is removed and the antigen is adsorbed to fumed silica at slightly elevated pH and lowered temperature. The antigen is then eluted from the fumed silica, treated to remove residual silica, concentrated, diafiltered and sterile filtered.

The following example illustrates the present invention without, however, limiting the same thereto.

## EXAMPLE

pHBpreSGAP347/19T (described in European patent application 0 174 444 as pHBpreS56GAP347/33) was used to construct an expression cassette, as described previously [Kniskern et al., Gene 46:135-141, (1986)], which was composed of: (a) ca. 1050 bp of the GAP491 promoter, (b) a 10 bp yeast-derived flanking sequence, (c) 846 base pairs of the HBV preS2 + S gene (serotype adw) lacking any viral flanking sequences, and (d) ca. 350 bp of the yeast ADH1 terminator. This expression cassette was inserted into the yeast shuttle vector pCl/1 [Beggs, Nature 275:104 (1978); Rosenberg et al., Nature 312:77, (1984)] and used to transform yeast strain CF42 (generating transformant pYGpreS2S-1) parallel experiment, the plasmid pHBpreSGAP347/19T. Parental strain CF42 was obtained as follows: a spontaneous ura3 mutation in yeast strain 2150-2-3 (gift of L. Hartwell. U. of Washington) was selected (Boeke et al., Mol. Gen. Genet. 197:345-346, 1984). The resulting strain (MATa, ade1, leu2-04, ura3, cir°) was diploidized by transforming with plasmid YCp50-HO [Jensen et al., P.N.A.S. USA 80:3035-3039(1983)]. The functional yeast gene HO allows cells to switch mating type. Thus, progeny from single cell transformants will be a mixture of both "a" and "α" mating types and will mate during colony growth. A diploid clonal isolate was cured of the plasmid and designated CF42, (MATa/α, ade1, leu2-04, ura3). These transformants were established as frozen stocks for evaluation and subsequent experimentation.

Recombinant yeast from the above frozen stocks are grown in YEHD medium. After growth to stationary phase, yeast cells are broken.

To an 8 ml slurry of broken yeast cells of strain CF42 containing 0.5% of Triton X-100 there is added 1 ml PBS (phosphate buffered saline), 0.7 g $K_2HPO_4$, 1 ml 5 M NaCl, 6 ml of a 20% solution of PEG 3350 and 4 ml of a 20% solution of Dextan T500. The resulting mixture is centrifuged at 2500 x g at 4°C for 15 minutes.

The antigen-containing PEG 3350 top phase (12-13 ml) is separated and diluted 1:2 with PBS and filtered through a 0.2 µ filter. The filtrate is concentrated and diafiltered with 10 volumes PBS to remove the PEG 3350 on an Amicon $10^5$ mw cutoff membrane. Residual Triton X-100 is removed with XAD-2 resin and the resin beads are then separated by filtration.

The filtrate is adsorbed to Aerosil 380 (having a surface of 380 $m^2$/g) at a pH of 7.5-7.6 and a temperature of 2-8°C for a period of 2.5 hours and then centrifuged at 2500 x g at 4°C for 15 minutes. The amount of Aerosil 380 that is used is determined from the formula: Weight (gm) Aerosil = 0.0259 x (Filtrate Vol In M1) x $(A_{280}-A_{350})$ where $(A_{280}-A_{350})$ is the difference in absorbance at $\lambda_{280}$ and $\lambda_{350}$ of a 1:10 dilution of the filtrate. Therefore, the amount of Aerosil 380 added will be a function of cell breakage as well as removal of waste proteins, etc., during processing.

The antigen is eluted from the Aerosil 380 by the addition of 15 ml of borate buffer, pH 8.7, per g of Aerosil 380. This elution step is repeated once and the combined eluates are centrifuged 30 minutes at 11,000 xg to remove Aerosil 380 fines, diafiltered with borate buffer on an Amicon $10^5$ mw cutoff membrane, and sterile filtered through a 0.22 µ filter. The antigen to protein ratio of preS2 + S antigen in the filtrate in replicate runs was from 12-20% with antigen yields of from 30-65% based on total antigen indicating no significant degradation during processing.

## Claims

1. A method of isolating yeast-produced preS2 + S antigen from a liquid phase comprising:
forming the liquid phase into an upper preS2 + S antigen-containing hydrophobic phase and a lower hydrophilic phase,
separating the upper phase from the lower phase,
adsorbing the antigen in the antigen containing phase on fumed silica,
and desorbing the adsorbed antigen.

2. A method according to claim 1 wherein the desorbed antigen is diafiltered.

3. A method according to claim 2 wherein the antigen is diafiltered against borate buffer.

4. A method according to claim 2 wherein the antigen is sterile filtered following diafiltration.

5. A method of isolating yeast-produced preS2 + S antigen comprising desorbing the antigen from fumed silica to which it was adsorbed from a hydrophobic aqueous phase, and recovering antigen without significant degradation in a yield of from about 30% to about 65%.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | NUCLEIC ACIDS RESEARCH, vol. 11, no. 9, 1983, pages 2745-2763, IRL Press Ltd, Oxford, GB; R.A. HITZEMAN et al.: "Expression of hepatitis B virus surface antigen in yeast" * Page 2748, lines 10-17; page 2757, line 9 - page 2758, line 8 * | 1-5 | C 12 P 21/02 A 61 K 39/29 // C 12 N 15/00 |
| Y | EP-A-0 155 007 (JURIDICAL FOUNDATION THE CHEMOSERO THERAPEUTIC INSTITUTE) * Page 8, line 11 - page 11, line 20 * | 1-5 | |
| A | FR-A-2 600 341 (OSAKA UNIVERSITY) * Page 7, line 9 - page 8, line 27; page 14, line 26 - page 16, line 1 * | 1 | |
| A | EP-A-0 135 435 (MERCK & CO., INC.) * Page 11, example 2 * | 1 | |
| A | PROCEEDINGS OF THE NATIONAL ACADEMY OF SCIENCES OF THE USA, vol. 82, no. 20, October 1985, pages 6830-6834, Washington, US; D.E. WAMPLER et al.: "Multiple chemical forms of hepatitis B surface antigen produced in yeast" * Page 6830, column 2, lines 26-38 * | 1 | TECHNICAL FIELDS SEARCHED (Int. Cl.4) C 12 N C 12 P A 61 K |
| D,A | EP-A-0 174 444 (CHIRON CORP.) | | |

The present search report has been drawn up for all claims

| Place of search | Date of completion of the search | Examiner |
|---|---|---|
| THE HAGUE | 13-01-1989 | SKELLY J.M. |